# EUROPEAN PATENT APPLICATION

(11) **EP 3 184 647 A1**
(43) Date of publication of application: **28.06.2017**
(21) Application number: 15202494.9
(22) Date of filing: 23.12.2015
(51) Int. Cl.: C12Q 1/68

(54) **LASER PCR AND BEAD DETECTION IN ONE REACTION CHAMBER**

(71) Applicant: Koninklijke Philips N.V., 5656 AE Eindhoven (NL)
(72) Inventor: The designation of the inventor has not yet been filed
(74) Representative: de Haan, Poul Erik

(57) **Abstract**

The present invention refers to a method for amplification and optical detection of nucleic target sequences comprising the steps of electromagnetic bead excitation PCR amplification using nanoparticles functionalized with an oligonucleotide primer and optical detection of the amplified nucleic acid sequence hybridized to a detection surface.

## Description

### FIELD OF THE INVENTION

The present invention refers to a method for amplification and optical detection of nucleic target sequences comprising the steps of electromagnetic bead excitation PCR amplification using nanoparticles functionalized with an oligonucleotide primer and optical detection of the amplified nucleic acid sequence hybridized to a detection surface.

### BACKGROUND OF THE INVENTION

Techniques for the detection and amplification of extremely small quantities of nucleic acid are an indispensable tool in modern molecular biology and biochemical research and are e.g. used for the diagnosis and detection of diseases, in forensic science, DNA sequencing and recombinant DNA technology.

The advent of microfluidics allows the handling low amounts of sample.

### SUMMARY OF THE INVENTION

Thus there is a need for efficient methods that allow for amplification and optical detection of nucleic target sequences with high sensitivity. It would be desirable that the method for amplification and optical detection is fast, that means that the amplification and detection process can be completed in a short time, i.e. within less than 15 minutes.

In addition, it would by desirable that during the PCR amplification heating of the complete solution or the complete reaction vessel is avoided.

In particular, it would be advantageous to integrate PCR amplification into a cartridge and/or microfluidic device, without the drawbacks of pressure build-up and condensation during heating.

In addition it would be advantageous that the PCR amplification and target sequence detection can be carried out by the same device. In particular it would be advantageous that the PCR amplification and target sequence detection of the target sequence occur within the same cartridge and/or microfluidic device. It would be also desirable that the PCR amplification and target sequence detection can be carried out within the same reaction vessel.

Further it would be advantageous to minimize the amount of compounds, i.e. enzymes for the amplification reaction.

Also, it would be desirable to provide methods for amplification and optical detection of nucleic target sequences with less liquid transfer and/or less complex microfluidics. To better address these desire amplification and detection are carried out in one chamber.

To better address one or more of these concerns, the invention provides a method for amplification and optical detection of a nucleotide target sequence comprising the following steps:
(a) electromagnetic bead excitation PCR amplification of the nucleotide target sequence using nanoparticles functionalized with an oligonucleotide primer which is complementary to at least part of the target sequence to generate an amplified product;
(b) hybridization of the amplified product to a detection surface functionalized with an oligonucleotide probe complementary to at least part of the target sequence;
(c) measuring the amplified product by optically detecting optically detectable particles bound to the amplified product at the detection surface;
wherein the amplified product is allowed to bind to the optically detectable particles during or after the photothermal PCR amplification.

In some embodiments the electromagnetic bead excitation PCR is photothermal PCR and the nanoparticles are photothermal particles.

In some embodiments the optically detectable particles are magnetic particles and step (b) further comprises moving the magnetic particles to the detection surface using magnetic forces.

In some embodiments steps (a) to (c) are carried out in the same reaction chamber, wherein the reaction chamber is preferably part of a microfluidic cartridge.

Usually in step (a) the photothermal particles are excited to generate heat, preferably by exposure to laser light.

The photothermal particles may have a size of 5 nm to 150 nm, preferably of 10 nm to 100 nm, more preferably of 20 nm to 90 nm and most preferably of 30 nm to 80 nm. Preferably the photothermal particles may be gold particles.

Typically, the detecting in step (c) is performed using frustrated total internal reflection (FTIR).

In some embodiments the oligonucleotide probe of the detection surface is blocked at the 3'-end.

In a specific embodiment step (a) comprises the following steps:
(i) contacting the nucleotide target sequence with
   - photothermal particles functionalized with a first oligonucleotide primer which is complementary to at least part of the target sequence, and
   - a second oligonucleotide primer which is complementary to at least part of the target sequence;
(ii) allowing amplification of the target sequence in order to generate the amplified product;
(iii) exciting the photothermal particles to generate heat;
wherein the amplified product is allowed to bind to optically detectable particles by means of hybridization to an oligonucleotide probe which is complementary to at least part of the target sequence and with which the optically detectable particles are functionalized.

In other embodiments, step (a) comprises the following steps:
(i) contacting the nucleotide target sequence with
   - photothermal particles functionalized with a first oligonucleotide primer which is complementary to at least part of the target sequence, and
   - a second oligonucleotide primer which is complementary to at least part of the target sequence;
(ii) allowing amplification of the target sequence in order to generate the amplified product;
(iii) exciting the photothermal particles to generate heat;
and wherein the amplified product is allowed to bind to optically detectable particles by means of hybridization to an oligonucleotide probe which is complementary to at least part of the target sequence and with which the optically detectable particles are functionalized;
In further embodiments, step (a) comprises the following steps:
(i) contacting the nucleotide target sequence with photothermal particles functionalized with a first oligonucleotide primer which is complementary to at least part of the target sequence, and optically detectable particles functionalized with a second oligonucleotide primer which is complementary to at least part of the target sequence;
(ii) allowing amplification of the target sequence in order to generate the amplified product;
(iii) exciting the photothermal particles to generate heat;

In some embodiments step (a) comprises the following steps:
(i) contacting the nucleotide target sequence with photothermal particles which are optically detectable and are functionalized with an oligonucleotide primer which is complementary to at least part of the target sequence;
(ii) allowing amplification of the target sequence in order to generate the amplified product;
(iii) exciting the photothermal particles to generate heat;

Other embodiments refer to a method in which step (a) comprises the following steps:
(i) contacting the nucleotide target sequence with photothermal particles functionalized with a first oligonucleotide primer which is complementary to at least part of the target sequence and functionalized with a first binding molecule, and optionally a second oligonucleotide primer which is complementary to at least part of the target sequence;
(ii) allowing amplification of the target sequence in order to generate the amplified product;
(iii) exciting the photothermal particles to generate heat;
(iv) contacting the photothermal particles with optically detectable particles functionalized with a second binding molecule capable of binding the first binding molecule of the photothermal particles;

Typically step (a) is repeated for 1 to 100 cycles, preferably for 1 to 50 cycles, more preferably for 1 to 40 cycles, even more preferably for 1 to 30 cycles. Steps (b) and/or (c) may be repeated or may not be repeated. Steps (b) and/or (C) may be repeated with the same or with different frequencies compared to step (a). Typically the frequency of step (a) is higher than the frequency of step (c).

### BRIEF DESCRIPTION OF THE DRAWINGS

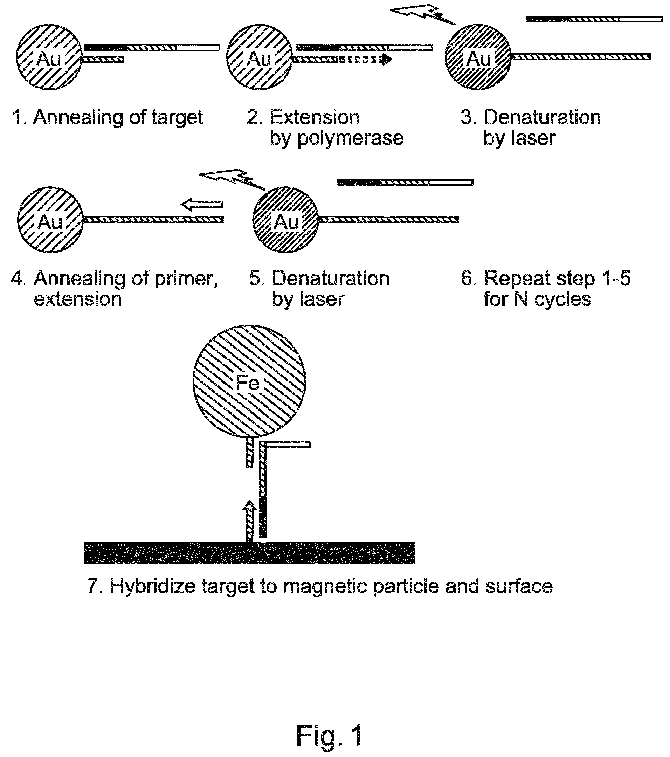

Fig 1 depicts an endpoint detection assay, .i.e. photothermal PCR amplification steps 1 to 5 are completed before the detection (step 7) takes place.
   The nucleotide target sequence is contacted with gold particles functionalized with a first oligonucleotide primer which is complementary to part of the target sequence and further contacted with a second oligonucleotide primer which is complementary to part of the target sequence; The amplified product hybridizes to optically detectable particles via an oligonucleotide probe complementary to part of the target sequence and hybridizes to the detection surface via an oligonucleotide primer blocked at the 3'end which is complementary to part of the target sequence. Fe: magnetic particles; Au: gold particles.

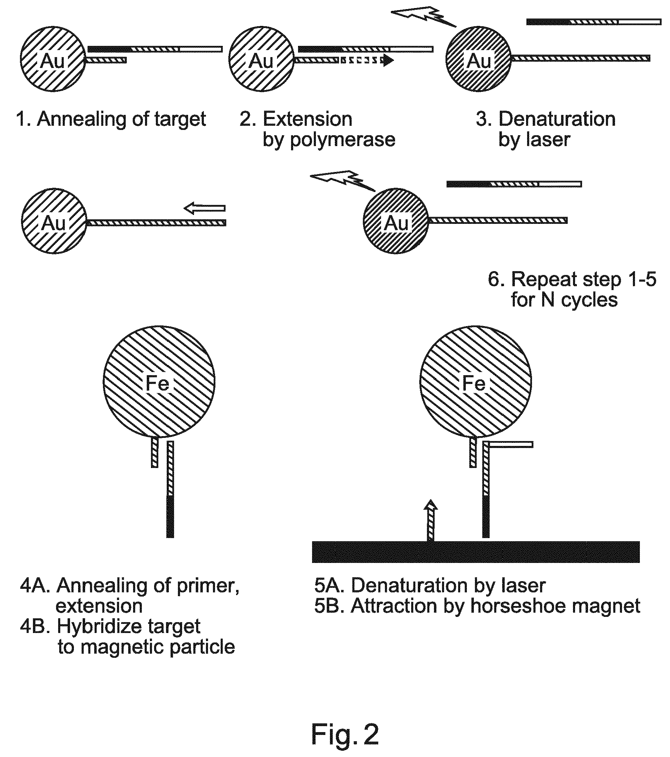

Fig. 2 depicts a real time detection assay i.e. detection takes place after each amplification cycle. The nucleotide target sequence is contacted with gold particles functionalized with a first oligonucleotide primer which is complementary to part of the target sequence and further contacted with a second oligonucleotide primer which is complementary to at least part of the target sequence; The amplified product hybridizes to optically detectable particles via an oligonucleotide probe complementary to part of the target sequence and hybridizes to the detection surface via an oligonucleotide primer blocked at the 3'end which is complementary to part of the target sequence. Fe: magnetic particles; Au: gold particles.

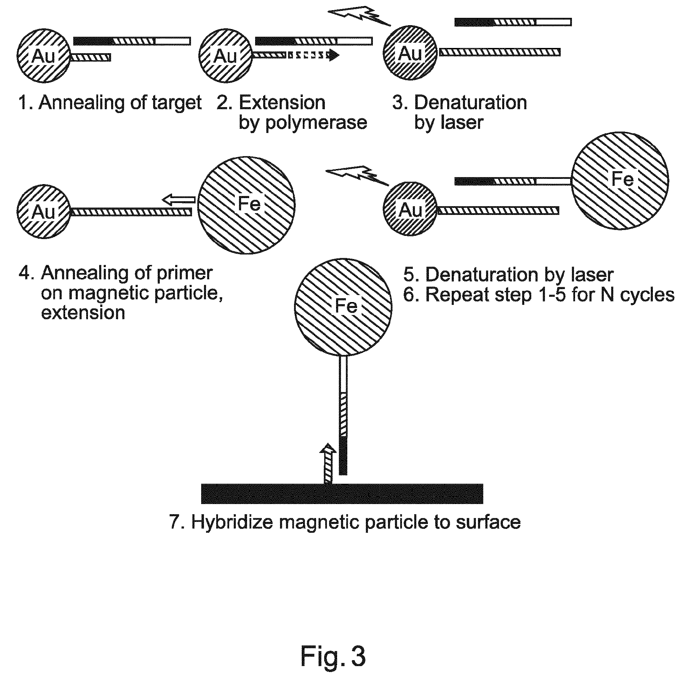

Fig. 3 shows an embodiment in which the attachment of the amplified product occurs during the PCR amplification reaction via elongation of a primer attached to the magnetic particle. In this embodiment the gold particles are functionalized with a primer complementary to a first region in the target sequence and the magnetic particles are functionalized by a primer which is complementary to a second region in the target sequence. By extension of the primer the amplified target sequence is bound to the magnetic particle. The gold particles are employed for photothermal PCR amplification and the magnetic particles are employed for optical detection. Fe: magnetic particles; Au: gold particles.

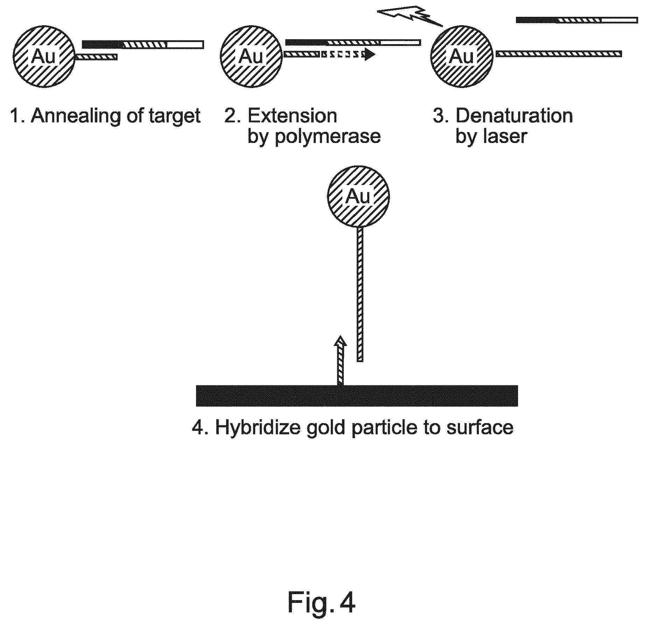

Fig. 4 depicts an embodiment in which the amplified product is allowed to bind to the optically detectable particles during photothermal PCR amplification. In this embodiment gold particles are functionalized by a primer which is complementary to the target sequence. By extension of the primer the amplified target sequence is bound to the gold particle. The gold particles are employed for photothermal PCR amplification and optical detection. Au: gold particles.

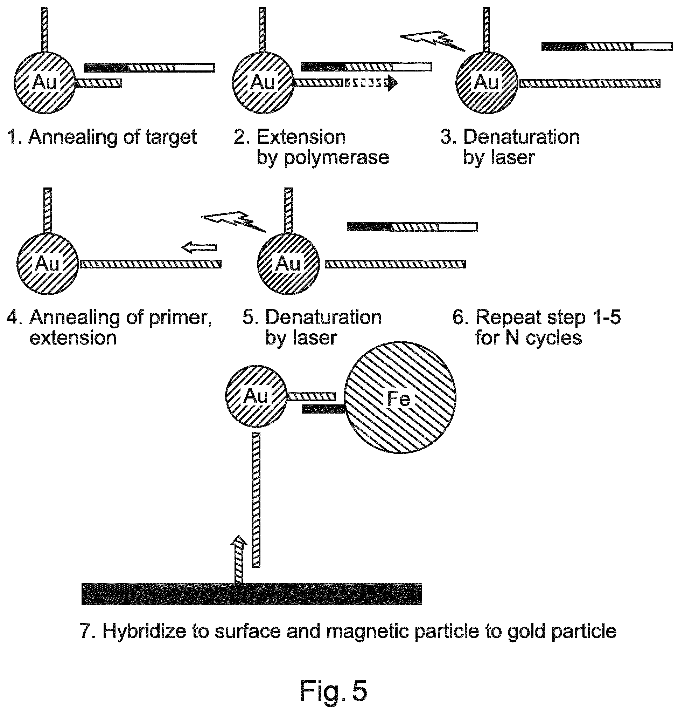

Fig. 5 shows an embodiment in which the gold particles are functionalized with a primer which is complementary to the target sequence and functionalized with a second binding molecule which is capable of binding the first binding molecule of the photothermal particles. Fe: magnetic particles; Au: gold particles.

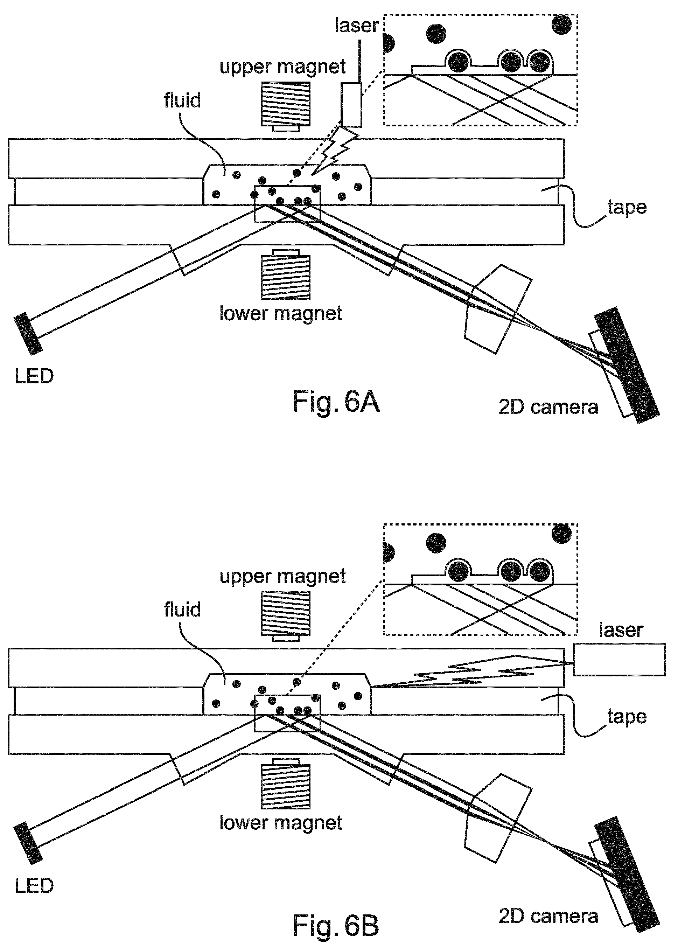

Fig. 6 shows possible arrangements allowing detection and laser heating in the same chamber. In Fig. 6A the laser for heating is exposed to the amplification and detection chamber from top and the laser for detection is exposed said camber from below. In Fig. 6B the laser for heating is exposed to the amplification and detection chamber from the side and the laser for detection is exposed said camber from below. Fe: magnetic particles; Au: gold particles.

### DETAILED DESCRIPTION OF EMBODIMENTS

Although the present invention will be described with respect to particular embodiments, this description is not to be construed in a limiting sense.

Before describing in detail exemplary embodiments of the present invention, definitions important for understanding the present invention are given.

As used in this specification and in the appended claims, the singular forms of "a" and "an" also include the respective plurals unless the context clearly dictates otherwise.

In the context of the present invention, the terms "about" and "approximately" denote an interval of accuracy that a person skilled in the art will understand to still ensure the technical effect of the feature in question. The term typically indicates a deviation from the indicated numerical value of ±20 %, preferably ±15 %, more preferably ±10 %, and even more preferably ±5 %.

It is to be understood that the term "comprising" is not limiting. For the purposes of the present invention the term "consisting of" is considered to be a preferred embodiment of the term "comprising of'. If hereinafter a group is defined to comprise at least a certain number of embodiments, this is meant to also encompass a group which preferably consists of these embodiments only.

Furthermore, the terms "first", "second", "third" or "(a)", "(b)", "(c)", "(d)" etc. and the like in the description and in the claims, are used for distinguishing between similar elements and not necessarily for describing a sequential or chronological order. It is to be understood that the terms so used are interchangeable under appropriate circumstances and that the embodiments of the invention described herein are capable of operation in other sequences than described or illustrated herein.

In case the terms "first", "second", "third" or "(a)", "(b)", "(c)", "(d)" etc. relate to steps of a method or use there is no time or time interval coherence between the steps, i.e. the steps may be carried out simultaneously or there may be time intervals of seconds, minutes, hours, days, weeks, months or even years between such steps, unless otherwise indicated in the application as set forth herein below.

The terms "nucleic acid" or "nucleic acid molecule" refer to a deoxyribonucleotide or ribonucleotide polymer in either single-or double-stranded form, and also encompass known analogues of natural nucleotides that can function in a similar manner as naturally occurring nucleotides.

It is to be understood that this invention is not limited to the particular methodology, protocols, reagents etc. described herein as these may vary. It is also to be understood that the terminology used herein is for the purpose of describing particular embodiments only, and is not intended to limit the scope of the present invention that will be limited only by the appended claims. Unless defined otherwise, all technical and scientific terms used herein have the same meanings as commonly understood by one of ordinary skill in the art.

One aspect of the invention refers to a method for amplification and optical detection of a nucleotide target sequence comprising the following steps:
(a) electromagnetic bead excitation PCR amplification of the nucleotide target sequence using nanoparticles functionalized with an oligonucleotide primer which is complementary to at least part of the target sequence to generate an amplified product;
(b) hybridization of the amplified product to a detection surface functionalized with an oligonucleotide probe complementary to at least part of the target sequence;
(c) measuring the amplified product by optically detecting optically detectable particles bound to the amplified product at the detection surface;
wherein the amplified product is allowed to bind to the optically detectable particles during or after the photothermal PCR amplification.

In some embodiments the electromagnetic bead excitation PCR amplification of the nucleotide target sequence is photothermal PCR and the nanoparticles a photothermal particles.

Therefore, one embodiment of the invention refers to a method for amplification and optical detection of a nucleotide target sequence comprising the following steps:
(a) photothermal PCR amplification of the nucleotide target sequence using photothermal particles functionalized with an oligonucleotide primer which is complementary to at least part of the target sequence to generate an amplified product;
(b) hybridization of the amplified product to a detection surface functionalized with an oligonucleotide probe complementary to at least part of the target sequence;
(c) measuring the amplified product by optically detecting optically detectable particles bound to the amplified product at the detection surface;
wherein the amplified product is allowed to bind to the optically detectable particles during and/or after the photothermal PCR amplification.

The term "nucleotide target sequence" refers to a nucleic acid. The nucleic acid may be derived from a biological sample. It is recognized that the target nucleic acids can be derived from essentially any source of nucleic acids (e.g., including, but not limited to chemical syntheses, amplification reactions, forensic samples, etc.) A nucleic target sequence may e.g. be a gene, DNA, cDNA, RNA, mRNA or fragments thereof. The presence or absence of one or more target nucleic acids may be detected, or the amount of one or more target nucleic acids may be quantified by the methods disclosed herein. Nucleotide target sequences(s) preferentially have nucleotide sequences that are complementary to the nucleic acid sequences of the corresponding oligonucleotide primer and/or oligonucleotide probe used for amplification and detection in method described herein, respectively.

The term "complementary" as used herein means that only matching bases are present. The term complementary "to at least part of the target sequence" as used herein means that at a sequence (such as a primer or a probe) comprises a sequence that is complementary to at least 5 nucleotides of the target sequence. Also included are sequences which are complementary to the complete target sequence. Typically, "to at least part of the target sequence" means 5 to 1000 nucleotides, preferably 7 to 500 nucleotides, more preferably 8 to 100 nucleotides, even more preferably 10 to 50 nucleotides, still more preferably 15 to 30 nucleotides, most preferably 18 to 22 nucleotides.

The term "electromagnetic excitation PCR" as used herein refers to a PCR amplification method in which the temperature changes for the PCR reaction cycles is employed by exciting nanoparticles with electromagnetic radiation. The term "photothermal PCR amplification", as used herein refers to a PCR amplification method in which the temperature changes for the PCR reaction cycles is employed by exciting the photothermal particles with electromagnetic radiation, in particular excitation by light (visible and non-visible), more particularly by laser light. Upon decreasing or switching off the electromagnetic radiation, the nanoparticles cool down to the temperature of the surrounding solution. The surrounding solution remains at a constant temperature.

The skilled person is aware of temperature rages suitable for PCR reaction. The temperature for dehybridization and hybridization depend on the sequence of the nucleotide molecule, for example the target sequence and the primer. Typical dehybridization of the nucleotide strands occurs in a rage of 55°C to 95°C, preferably in a range between 60°C to 80°C depending on the nucleotide molecule. The temperature of the surrounding solution is selected so that hybridization and polymerization can be carried out and depends on the nucleotides and polymerase used in the reaction. Typically hybridization of the primers and polymerization occurs at 25°C to 80 °C, preferably at 40°C to 70 °C:

By excitation of the nanoparticle, the local temperature of the DNA molecule that is bound to the nanoparticle is increased leading to the dehybridization of the complementary nucleotide strands of a double strand nucleotide molecule. That means that in step (iii), i.e. exciting the photothermal particles to generate heat, the unbound nucleotide strand (i.e. the nucleotide strand that is hybridized to the nucleotide strand which is bound to the nanoparticle) is released. When the electromagnetic radiation (i.e. laser light) is decreased or switched of and the nanoparticle cools down, the DNA molecules attached to the bead are also cooling down to the temperature of the surrounding solution.

The term "electromagnetic excitation" includes for example radio waves, micro waves, X rays and light (visible and non visible), in particular laser light. Thus the present invention is not limited to the excitation of photothermal particles by light, but includes also the excitation of nanoparticles by other electromagnetic sources, such as radio waves, micro waves an X rays.

Typically the nanoparticles are excited by light, for example by light emitted by light emitting diodes, flash lamps or lasers. Usually the nanoparticles are excited by laser light. Light in the context of the invention is understood to include the spectrum of electromagnetic radiation from far infrared to far ultraviolet.

The term "oligonucleotide probe" as used herein refers to a specific oligonucleotide sequence which is capable of hybridizing to a target nucleic acid sequence due to its complementarity. Typically such oligonucleotide probes will have a length of about 10 to about 1000 nucleotides, of about 10 to about 800 nucleotides, of about 10 to about 700 nucleotides, of about 10 to about 600 nucleotides, of about 10 to about 500 nucleotides, of about 15 to about 400 nucleotides, of about 15 to about 300 nucleotides, of about 15 to about 200 nucleotides, about 20 to about 150 nucleotides, about 20 to about 100 nucleotides, of about 20 to about 90 nucleotides, of about 20 to about 80 nucleotides, about 20 to about 70 nucleotides, about 20 to about 60 nucleotides or of about 20 to about 50 nucleotides. Typically, oligonucleotide probes will have a length of about 20, 30, 40, 50, 60, 70 nucleotides. Usually the oligonucleotide probe is block at the 3'-end. This has the advantage that the probe cannot take part in the PCR amplification process.

The term "oligonucleotide primer" as used herein refers to a specific oligonucleotide sequence which is capable of hybridizing to a target nucleic acid sequence due to its complementarity and providing a start molecule for the amplification of the target molecule to which the primer has hybridized. Typically such oligonucleotide primers will have a length of about 10 to about 1000 nucleotides, of about 10 to about 800 nucleotides, of about 10 to about 700 nucleotides, of about 10 to about 600 nucleotides, of about 10 to about 500 nucleotides, of about 15 to about 400 nucleotides, of about 15 to about 300 nucleotides, of about 15 to about 200 nucleotides, about 20 to about 150 nucleotides, about 20 to about 100 nucleotides, of about 20 to about 90 nucleotides, of about 20 to about 80 nucleotides, about 20 to about 70 nucleotides, about 20 to about 60 nucleotides or of about 20 to about 50 nucleotides. Typically, oligonucleotide primers will have a length of about 20, 30, 40, 50, 60, 70 nucleotides.

The oligonucleotide may be DNA, RNA, PNA, CNA, HNA, LNA or ANA. The DNA may be in the form of, e.g. A-DNA, B-DNA or Z-DNA. The RNA may be in the form of, e.g. p-RNA, i.e. pyranosysl-RNA or structurally modified forms like hairpin RNA or a stem-loop RNA.

The term "PNA" relates to a peptide nucleic acid, i.e. an artificially synthesized polymer similar to DNA or RNA which is used in biological research and medical treatments, but which is not known to occur naturally. The PNA backbone is typically composed of repeating N-(2-aminoethyl)-glycine units linked by peptide bonds. The various purine and pyrimidine bases are linked to the backbone by methylene carbonyl bonds. PNAs are generally depicted like peptides, with the N-terminus at the first (left) position and the C-terminus at the right.

The term "CNA" relates to an aminocyclohexylethane acid nucleic acid. Furthermore, the term relates to a cyclopentane nucleic acid, i.e. a nucleic acid molecule comprising for example 2'-deoxycarbaguanosine.

The term "HNA" relates to hexitol nucleic acids, i.e. DNA analogues which are built up from standard nucleobases and a phosphorylated 1,5-anhydrohexitol backbone.

The term "LNA" relates to locked nucleic acids. Typically, a locked nucleic acid is a modified and thus inaccessible RNA nucleotide. The ribose moiety of an LNA nucleotide may be modified with an extra bridge connecting the 2' and 4' carbons. Such a bridge locks the ribose in a 3'-endo structural conformation. The locked ribose conformation enhances base stacking and backbone pre-organization. This may significantly increase the thermal stability, i.e. melting temperature of the oligonucleotide.

The term "ANA" relates to arabinoic nucleic acids or derivatives thereof. A preferred ANA derivative in the context of the present invention is a 2'-deoxy-2'-fluoro-beta-D-arabinonucleoside (2'F-ANA).

In a further preferred embodiment oligonucleotides may comprise a combination of any one of DNA, RNA, PNA, CNA, HNA, LNA and ANA. Particularly preferred are mixtures of LNA nucleotides with DNA or RNA bases.

The term "amplified product", as used herein, refers the products of the amplification of nucleic acids, using e.g. PCR or any other method suitable for the amplification of nucleic acids. In one embodiment, the length of an amplified product is between 50 and 800 bases, preferably between 50 and 400 bases and more preferably between 50 and 200 bases or 100 to 200 bases.

If a nucleic acid capture probe or any other nucleic acid molecule described herein is said to be "specific" for a target nucleic acid or any other nucleotide sequence or to "specifically" bind to a target nucleic acid or any other nucleotide sequence this refers to preferential binding, duplexing, or hybridizing of said capture probe or any other nucleic acid molecule to a particular nucleotide sequence under stringent conditions. The term "stringent conditions" refers to conditions under which a probe will hybridize preferentially to its target sequence, and to a lesser extent to, or not at all to, other sequences. Stringent conditions are sequence-dependent and will be different in different circumstances.

Stringent conditions in this context may for example be selected to be about 5°C lower than the thermal melting point (Tm) for the specific sequence at a defined ionic strength and pH. The Tm is the temperature (under defined ionic strength, pH, and nucleic acid concentration) at which 50% of the probes complementary to the target sequence hybridize to the target sequence at equilibrium. For example, stringent conditions may be those in which the salt concentration is at least about 0.01 to 1.0 M Na ion concentration (or other salts) at pH 7.0 to 8.3 and the temperature is at least about 30°C for short probes (e.g., 10 to 50 nucleotides). Stringent conditions may also be achieved with the addition of destabilizing agents such as formamide.

The term "quantifying" as used herein with regard to nucleic acid abundances or concentrations may refer to absolute or to relative quantification. Absolute quantification may e.g. be accomplished by inclusion of known concentration(s) of one or more target nucleic acids (control nucleic acids) and referencing the signal intensity of target nucleic acids of unknown concentration with the target nucleic acids of known concentration (e.g. through generation of a standard curve). Relative quantification can be accomplished, for example, by comparison of signals between two or more target nucleic acids.

The nanoparticle (to be excited) of the present invention is a particle that is capable of being excited by electromagnetic waves. The photothermal particle is a particle that is capable being excited by light. Nanoparticles useful for the present invention are for example described in O. Govonorov et al. (Generating heat with metal nanoparticles, nanotoday, 2007, 2(8):30-38) and in Jacobson et al. (Remote electronic control of DNA hybridization though inductive coupling to an attached metal nanocrystal antenna", Nature, 2002,415: 152.155).

The nanoparticle (to be excited), in particular the photothermal particle, may be for example a metal particle, for example a noble metal, such as gold and silver. Preferably the nanoparticle may be a gold particle. The nanoparticle may be composed of a single material or may be composed of a mixture of materials.

The nanoparticles, in particular the photothermal particle, may be contacted with the nucleic acid via diffusion in aqueous medium. The concentration of the nanoparticles may be between 0.01 nM to 1000 nM, optionally 0.1 and 100nM

The nanoparticles, in particular the photothermal particles, may have a size of 5 nm to 150 nm, preferably of 10 nm to 100 nm, more preferably of 20 nm to 90 nm and most preferably of 30 nm to 80 nm.

The amplified product may move to the detection surface for example by diffusion. The amplified product may diffuse to the detection surface in a state which is free, i.e. not bound to the optically detectable particle. Alternatively the amplified product may diffuse to the detection surface in a state which bound to the optically detectable particle.

In a specific embodiment, the optically detectable particles are magnetic particles. Then the optically detectable particles may be moved to the detection surface by magnetic forces. The magnetic forces may be a magnetic field that is applied to the detection surface. The magnetic field may be generated for example in order to move the magnetic particles to the detection surface. The magnetic particles may be tethered to the detection surface during the PCR procedure by applying a magnetic field that moves the magnetic particles to the detection surface. This may have the advantage that the magnetic particles do not interfere with the PCR amplification process. After the amplification process the magnetic particles may be released into the sample volume by changing the magnetic field in that the particles are moved into the sample volume and mix with the amplified product. Alternatively the optically detectable particles may be distributed in the sample volume, i.e. not be tethered to the detection surface during the PCR amplification.

Frustrated total internal reflection (FTIR) is an imaging method in which light originating from a material with a higher refractive index strikes an interface with a material of lower refractive index at an angle larger than a critical angle. Under these conditions, the beam is reflected by the interface and an evanescent field is generated at the interface. If no particles are present close to the detection surface the light is completely reflected. If, however, particles are bound to the detection surface, light from the evanescent field is scattered by the particles and the amount of light reflected by the surface is decreased. By measuring the intensity of the reflected light with an optical detector, it is possible to estimate the amount of specific molecules of interest present within the sample.

Thus, in a preferred embodiment, detecting hybridization is undertaken by measuring signals within a distance of about 100 nm to about 300 nm, preferably within 100 nm to 200 nm and most preferably within 100 nm to 150 nm from the surface of the substrate.

The term "functionalized" in the context of the invention refers to the covalent or non-covalent attachment of nucleotide molecules to particles, such as nanoparticles, photothermal particles and/or optically detectable particles. For example beads with reactive carboxylic acid groups (such as but not limited to MyOne™ Carboxylic Acid Dynabeads®; Invitrogen) can be functionalized with a oligonucleotide having a primary amino group attached by a linker (for example IDT®) to its 5' end (covalent attachment). Alternatively strepatavidin-biotin interaction can be used to immobilize oligonucleotides on beads.

The amplification process and the detection process may be carried out in different reaction chambers or in the in the same reaction chamber. In specific embodiments the amplification process and the detection process may be carried out in the same reaction chamber. This may be advantageous since this assembly results in less sample volume, less complex fluidic, less liquid transfer and a simple cartridge design. In a specific embodiment the reaction chamber is preferably part of a microfluidic cartridge.

These embodiments can be carried out as endpoint assays, i.e. optical detection of the detectable particles is carried out after all amplification steps have been completed. That means that step (c) is carried out after completion of all executions of steps (a) and (b). Alternatively step (c) and step (b) are carried out after completion of all executions of step (a). A specific embodiment of such endpoint assay is depicted in Figure 1. Alternatively, the assays may be carried out as real-time detection assay wherein the optical detection can occur after each amplification cycle. That means that step (a), step (b) and step (c) are carried out in consecutive order with the same frequency. A specific embodiment of such a real time assay is depicted in Figure 2. Another alternative is a semi-real-time assay which is an intermediate between the end point and the real-time assay. In the semi-real-time assay the optical detection frequency is slower than the amplification frequency. For example optical detection (step (c)) is carried out only after every second amplification cycle (step (a)). In certain embodiments optical detection occurs after every second, after every third, after every fourth, after every fifth, after every sixth, after every seventh, after every eighth, after every ninth, after every tenth, after every eleventh, after every twelfth, after every thirteenth, after every fourteenth, after every fifteenth, after every sixteenth, after every seventeenth, after every eighteenth, after every nineteenth, after every twentieth or after every *n*th execution of step (a). Step (b) may be carried out in the frequency of step (a) or in the frequency of step (c).

### 1. Binding of the amplified product to the optically detectable beads by means of hybridization

In some embodiments the amplified product is allowed to bind to the optically detectable particles by means of hybridization to an oligonucleotide probe which is complementary to at least part of the target sequence and with which the optically detectable particles are functionalized.

Typically, in these embodiments step (a) comprises the following steps:
(i) contacting the nucleotide target sequence with
   - photothermal particles functionalized with a first oligonucleotide primer which is complementary to at least part of the target sequence, and
   - a second oligonucleotide primer which is complementary to at least part of the target sequence;
(ii) allowing amplification of the target sequence in order to generate the amplified product;
(iii) exciting the photothermal particles to generate heat;
wherein the amplified product is allowed to bind to optically detectable particles by means of hybridization to an oligonucleotide probe which is complementary to at least part of the target sequence and with which the optically detectable particles are functionalized.

Thus, specific embodiments refer to methods for amplification and optical detection of a nucleotide target sequence comprising the following steps:
(a) photothermal PCR amplification of the nucleotide target sequence using photothermal particles functionalized with an oligonucleotide primer which is complementary to at least part of the target sequence to generate an amplified product, comprising the following steps:
   (i) contacting the nucleotide target sequence with
      - photothermal particles functionalized with a first oligonucleotide primer which is complementary to at least part of the target sequence, and
      - a second oligonucleotide primer which is complementary to at least part of the target sequence;
   (ii) allowing amplification of the target sequence in order to generate the amplified product;
   (iii) exciting the photothermal particles to generate heat;
(b) hybridization of the amplified product to a detection surface functionalized with an oligonucleotide probe complementary to at least part of the target sequence;
(c) measuring the amplified product by optically detecting optically detectable particles bound to the amplified product at the detection surface;
wherein the amplified product is allowed to bind to the optically detectable particles during and/or after the photothermal PCR amplification and wherein the amplified product is allowed to bind to optically detectable particles by means of hybridization to an oligonucleotide probe which is complementary to at least part of the target sequence and with which the optically detectable particles are functionalized

Step (a) maybe repeated for 1 to 100 cycles, preferably for 1 to 50 cycles, more preferably for 1 to 40 cycles, even more preferably for 1 to 30 cycles.

The method employing binding of the amplified product to the optically detectable beads by means of hybridization can be carried out as end point detection, real-time detection or semi real-time detection assay.

### 1.1 Endpoint measurement assays

Specific embodiments are directed to endpoint measurement assays comprising the following steps:
(a) photothermal PCR amplification of the nucleotide target sequence using photothermal particles functionalized with an oligonucleotide primer which is complementary to at least part of the target sequence to generate an amplified product, comprising the following steps:
   (i) contacting the nucleotide target sequence with
      - photothermal particles functionalized with a first oligonucleotide primer which is complementary to at least part of the target sequence, and
      - a second oligonucleotide primer which is complementary to at least part of the target sequence;
   (ii) allowing amplification of the target sequence in order to generate the amplified product;
   (iii) exciting the photothermal particles to generate heat;
(b) hybridization of the amplified product to a detection surface functionalized with an oligonucleotide probe complementary to at least part of the target sequence;
(c) measuring the amplified product by optically detecting optically detectable particles bound to the amplified product at the detection surface;
wherein the amplified product is allowed to bind to the optically detectable particles during and/or after the photothermal PCR amplification and wherein the amplified product is allowed to bind to optically detectable particles by means of hybridization to an oligonucleotide probe which is complementary to at least part of the target sequence and with which the optically detectable particles are functionalized;
wherein step (a) is repeated for 1 to 100 cycles, preferably for 1 to 50 cycles, more preferably for 1 to 40 cycles, even more preferably for 1 to 30 cycles and wherein step (c) carried out once after the all repetitions of step (a) and step (b) are completed or alternatively wherein step (b) and (c) are carried out once after all repetition of step (a) are completed.

A specific embodiment for an endpoint assay is shown in Figure 1. In this embodiment the amplified product is allowed to bind to the optically detectable magnetic particles during the photothermal PCR amplification. This embodiment comprises the following steps:
(a) photothermal PCR amplification of the nucleotide target sequence using gold particles functionalized with an oligonucleotide primer which is complementary to at least part of the target sequence to generate an amplified product, comprising the following steps:
   (i) contacting the nucleotide target sequence with
      - gold particles functionalized with a first oligonucleotide primer which is complementary to at least part of the target sequence, and
      - a second oligonucleotide primer which is complementary to at least part of the target sequence;
   (ii) allowing amplification of the target sequence in order to generate the amplified product;
   (iii) exciting the gold particles to generate heat;
   (iv) allowing the amplified product to bind to optically detectable magnetic particles by means of hybridization to an oligonucleotide probe which is complementary to at least part of the target sequence and with which the optically detectable magnetic particles are functionalized;
(b) hybridization of the amplified product to a detection surface functionalized with an oligonucleotide probe complementary to at least part of the target sequence; wherein the magnetic particles to which the amplified product is bound are moved to the detection surface using magnetic forces.
(c) measuring the amplified product by optically detecting optically detectable magnetic particles bound to the amplified product at the detection surface; wherein steps (a) and (b) are repeated for 1 to 100 cycles, preferably for 1 to 50 cycles, more preferably for 1 to 40 cycles, even more preferably for 1 to 30 cycles and wherein step (c) is not repeated and carried out after the all repetitions of step (a) and step (b) are completed.

### 1.2 Real-time measurement assay

Specific embodiments are directed to real-time measurement assays, i.e. a method for amplification and optical detection of a nucleotide target sequence comprising the following steps:
(a) photothermal PCR amplification of the nucleotide target sequence using photothermal particles functionalized with an oligonucleotide primer which is complementary to at least part of the target sequence to generate an amplified product, comprising the following steps:
   (i) contacting the nucleotide target sequence with
      - photothermal particles functionalized with a first oligonucleotide primer which is complementary to at least part of the target sequence, and
      - a second oligonucleotide primer which is complementary to at least part of the target sequence;
   (ii) allowing amplification of the target sequence in order to generate the amplified product;
   (iii) exciting the photothermal particles to generate heat;
   (iv) allowing the amplified product to bind to optically detectable particles by means of hybridization to an oligonucleotide probe which is complementary to at least part of the target sequence and with which the optically detectable particles are functionalized.
(b) hybridization of the amplified product to a detection surface functionalized with an oligonucleotide probe complementary to at least part of the target sequence;
(c) measuring the amplified product by optically detecting optically detectable particles bound to the amplified product at the detection surface;
wherein steps (a) to (c) are repeated for 1 to 100 cycles, preferably for 1 to 50 cycles, more preferably for 1 to 40 cycles, even more preferably for 1 to 30 cycles.

An exemplary embodiment of a real-time assay is shown in Figure 2 and refers to a method for amplification and optical detection of a nucleotide target sequence comprising the following steps:
(a) photothermal PCR amplification of the nucleotide target sequence using gold particles functionalized with an oligonucleotide primer which is complementary to at least part of the target sequence to generate an amplified product;
   wherein step (a) comprises the following steps:
   (i) contacting the nucleotide target sequence with
      - gold particles functionalized with a first oligonucleotide primer which is complementary to at least part of the target sequence, and
      - optically detectable magnetic particles functionalized with a second oligonucleotide primer which is complementary to at least part of the target sequence;
   (ii) allowing amplification of the target sequence in order to generate the amplified product;
   (iii) exciting the gold particles to generate heat;
   (iv) allowing the amplified product to bind to optically detectable magnetic particles by means of hybridization to an oligonucleotide probe which is complementary to at least part of the target sequence and with which the optically detectable magnetic particles are functionalized;
(b) hybridization of the amplified product to a detection surface functionalized with an oligonucleotide probe complementary to at least part of the target sequence; wherein the magnetic particles to which the amplified product is bound are moved to the detection surface using magnetic forces;
(c) measuring the amplified product by optically detecting optically detectable magnetic particles bound to the amplified product at the detection surface; wherein steps (a) to (c) are repeated for 1 to 100 cycles, preferably for 1 to 50 cycles, more preferably for 1 to 40 cycles, even more preferably for 1 to 30 cycles.

### 1.3 Semi-Real-time measurement assay

Some embodiments refer to semi-real-time assays. In these assays measuring the amplified product is carried out more than once but at a lower frequency than the PCR amplification step. The hybridization step (b) may be carried out with the same frequency as step (a) or may be carried out with the same frequency as step (c). Step (c) measuring the amplified product may be carried out for example after every second, after every third, after every fourth, after every fifth, after every sixth, after every seventh, after every eighth, after every ninth, after every tenth, after every eleventh, after every twelfth, after every thirteenth, after every fourteenth, after every fifteenth, after every sixteenth, after every seventeenth, after every eighteenth, after every nineteenth, after every twentieth or after every *n*th execution of steps (a) and (b). Alternatively, steps (b) and (c) may be carried out for example after every 2nd, every 3rd, every 4th, every 6th, every 7th, every 8th, every 9th, every 10th or *n*th execution of step (a) and (b). These embodiments allow monitoring the reaction process and are very time efficient, since some of the detection steps are omitted. Embodiments referring to semi-real-time assays may comprise the following steps:
(a) photothermal PCR amplification of the nucleotide target sequence using photothermal particles functionalized with an oligonucleotide primer which is complementary to at least part of the target sequence to generate an amplified product, comprising the following steps:
   (i) contacting the nucleotide target sequence with
      - photothermal particles functionalized with a first oligonucleotide primer which is complementary to at least part of the target sequence, and
      - a second oligonucleotide primer which is complementary to at least part of the target sequence;
   (ii) allowing amplification of the target sequence in order to generate the amplified product;
   (iii) exciting the photothermal particles to generate heat;
(b) hybridization of the amplified product to a detection surface functionalized with an oligonucleotide probe complementary to at least part of the target sequence;
(c) measuring the amplified product by optically detecting optically detectable particles bound to the amplified product at the detection surface;
wherein the amplified product is allowed to bind to the optically detectable particles during and/or after the photothermal PCR amplification and wherein the amplified product is allowed to bind to optically detectable particles by means of hybridization to an oligonucleotide probe which is complementary to at least part of the target sequence and with which the optically detectable particles are functionalized;
wherein step (a) is repeated for 1 to 100 cycles, preferably for 1 to 50 cycles, more preferably for 1 to 40 cycles, even more preferably for 1 to 30 cycles and wherein step (c) is repeated with a lower frequency compared to step (a). Step (b) may be carried out in the frequency of step (a) or in the frequency of step (c).

Specific embodiments are directed to semi-real-time measurement assays comprise the following steps:
(a) photothermal PCR amplification of the nucleotide target sequence using photothermal particles functionalized with an oligonucleotide primer which is complementary to at least part of the target sequence to generate an amplified product, comprising the following steps:
   (i) contacting the nucleotide target sequence with
      - photothermal particles functionalized with a first oligonucleotide primer which is complementary to at least part of the target sequence, and
      - a second oligonucleotide primer which is complementary to at least part of the target sequence;
   (ii) allowing amplification of the target sequence in order to generate the amplified product;
   (iii) exciting the photothermal particles to generate heat;
   (iv) allowing the amplified product to bind to optically detectable particles by means of hybridization to an oligonucleotide probe which is complementary to at least part of the target sequence and with which the optically detectable particles are functionalized.
(b) hybridization of the amplified product to a detection surface functionalized with an oligonucleotide probe complementary to at least part of the target sequence;
(c) measuring the amplified product by optically detecting optically detectable particles bound to the amplified product at the detection surface;
wherein step (a) is repeated for 2 to 100 cycles, preferably for 2 to 50 cycles, more preferably for 2 to 40 cycles, even more preferably for 2 to 30 cycles, and wherein step (c) is repeated at a lower frequency compared to step (a). Step (b) may be carried out in the frequency of step (a) or in the frequency of step (c).

### 2. Binding of the amplified product to the optically detectable beads by extension of a primer with which the optically detectable beads are functionalized

In other specific embodiments the amplified product is allowed to bind to the optically detectable particles during the photothermal PCR amplification by extension of a primer with which the optically detectable beads are functionalized and which is complementary to at least part of the target sequence. In other words, step (a) of the method described herein comprises the following steps:
(i) contacting the nucleotide target sequence with
   - photothermal particles functionalized with a first oligonucleotide primer which is complementary to at least part of the target sequence, and
   - optically detectable particles functionalized with a second oligonucleotide primer which is complementary to at least part of the target sequence;
(ii) allowing amplification of the target sequence in order to generate the amplified product;
(iii) exciting the photothermal particles to generate heat;

Thus specific embodiments refer to methods comprising the following steps:
(a) photothermal PCR amplification of the nucleotide target sequence using photothermal particles functionalized with an oligonucleotide primer which is complementary to at least part of the target sequence to generate an amplified product; comprising the following steps:
   (i) contacting the nucleotide target sequence with
      - photothermal particles functionalized with a first oligonucleotide primer which is complementary to at least part of the target sequence, and
      - optically detectable particles functionalized with a second oligonucleotide primer which is complementary to at least part of the target sequence;
   (ii) allowing amplification of the target sequence in order to generate the amplified product;
   (iii) exciting the photothermal particles to generate heat;
(b) hybridization of the amplified product to a detection surface functionalized with an oligonucleotide probe complementary to at least part of the target sequence;
(c) measuring the amplified product by optically detecting optically detectable particles bound to the amplified product at the detection surface;
Step (a) may be repeated for 1 to 100 cycles, preferably for 1 to 50 cycles, more preferably for 1 to 40 cycles, even more preferably for 1 to 30 cycles.

The method employing optically detectable beads which are functionalized with a primer can be carried out as end point detection, real-time detection or semi real-time detection assay.

### 2.1 Binding of the amplified product to the optically detectable beads by extension of a primer with which the optically detectable beads are functionalized as end-point assay

An exemplary embodiment, in which the optically detectable beads are functionalized with a primer are used as an endpoint assay comprises the following steps:
(a) photothermal PCR amplification of the nucleotide target sequence using photothermal particles functionalized with an oligonucleotide primer which is complementary to at least part of the target sequence to generate an amplified product; comprising the following steps:
   (i) contacting the nucleotide target sequence with
      - photothermal particles functionalized with a first oligonucleotide primer which is complementary to at least part of the target sequence, and
      - optically detectable particles functionalized with a second oligonucleotide primer which is complementary to at least part of the target sequence;
   (ii) allowing amplification of the target sequence in order to generate the amplified product;
   (iii) exciting the photothermal particles to generate heat;
(b) hybridization of the amplified product to a detection surface functionalized with an oligonucleotide probe complementary to at least part of the target sequence;
(c) measuring the amplified product by optically detecting optically detectable particles bound to the amplified product at the detection surface;
wherein step (a) is repeated for 1 to 100 cycles, preferably for 1 to 50 cycles, more preferably for 1 to 40 cycles, even more preferably for 1 to 30 cycles and wherein step (c) is carried out once after all repetitions of step (a) and step (b) are completed or alternatively wherein steps (b) and (c) are carried out once after all repetitions of step (a) are completed.

A specific embodiment in which the optically detectable beads are functionalized with a primer are used in an endpoint assay is depicted in Figure 3. This embodiment comprises the following steps:
(a) photothermal PCR amplification of the nucleotide target sequence using gold particles functionalized with an oligonucleotide primer which is complementary to at least part of the target sequence to generate an amplified product; comprising the following steps:
   (i) contacting the nucleotide target sequence with
      - gold particles functionalized with a first oligonucleotide primer which is complementary to at least part of the target sequence, and
      - optically detectable magnetic particles functionalized with a second oligonucleotide primer which is complementary to at least part of the target sequence;
   (ii) allowing amplification of the target sequence in order to generate the amplified product;
   (iii) exciting the gold particles to generate heat;
   wherein steps (ii) and (iii) are repeated.
(b) hybridization of the amplified product to a detection surface functionalized with an oligonucleotide probe complementary to at least part of the target sequence;
(c) measuring the amplified product by optically detecting the optically detectable magnetic particles bound to the amplified product at the detection surface; wherein step (a) is repeated for 1 to 100 cycles, preferably for 1 to 50 cycles, more preferably for 1 to 40 cycles, even more preferably for 1 to 30 cycles and wherein steps (b) and (c) are carried out once after all repetitions of step (a) are completed.

### 2.2 Binding of the amplified product to the optically detectable beads by extension of a primer with which the optically detectable beads are functionalized as real-time assay

An exemplary embodiment, in which the optically detectable beads are functionalized with a primer are used as real-time assay comprises the following steps:
(a) photothermal PCR amplification of the nucleotide target sequence using photothermal particles functionalized with an oligonucleotide primer which is complementary to at least part of the target sequence to generate an amplified product; comprising the following steps:
   (i) contacting the nucleotide target sequence with
      - photothermal particles functionalized with a first oligonucleotide primer which is complementary to at least part of the target sequence, and
      - optically detectable particles functionalized with a second oligonucleotide primer which is complementary to at least part of the target sequence;
   (ii) allowing amplification of the target sequence in order to generate the amplified product;
   (iii) exciting the photothermal particles to generate heat;
(b) hybridization of the amplified product to a detection surface functionalized with an oligonucleotide probe complementary to at least part of the target sequence;
(c) measuring the amplified product by optically detecting optically detectable particles bound to the amplified product at the detection surface;
wherein steps (a) to (c) are repeated for 1 to 100 cycles, preferably for 1 to 50 cycles, more preferably for 1 to 40 cycles, even more preferably for 1 to 30 cycles.

### 2.3 Binding of the amplified product to the optically detectable beads by extension of a primer with which the optically detectable beads are functionalized as semi-real-time assay:

An exemplary embodiment, in which the optically detectable beads are functionalized with a primer are used as semi-real-time assay comprises the following steps:
(a) photothermal PCR amplification of the nucleotide target sequence using photothermal particles functionalized with an oligonucleotide primer which is complementary to at least part of the target sequence to generate an amplified product; comprising the following steps:
   (i) contacting the nucleotide target sequence with
      - photothermal particles functionalized with a first oligonucleotide primer which is complementary to at least part of the target sequence, and
      - optically detectable particles functionalized with a second oligonucleotide primer which is complementary to at least part of the target sequence;
   (ii) allowing amplification of the target sequence in order to generate the amplified product;
   (iii) exciting the photothermal particles to generate heat;
(b) hybridization of the amplified product to a detection surface functionalized with an oligonucleotide probe complementary to at least part of the target sequence;
(c) measuring the amplified product by optically detecting optically detectable particles bound to the amplified product at the detection surface;
wherein step (a) is repeated for 1 to 100 cycles, preferably for 1 to 50 cycles, more preferably for 1 to 40 cycles, even more preferably for 1 to 30 cycles and wherein step (c) is repeated with a lower frequency compared to step (a). Step (b) may be carried out in the frequency of step (a) or in the frequency of step (c).

### 3. Binding of the amplified product to the photothermal particles by extension of a primer with which the photothermal beads are functionalized and using said photothermal particles for optical detection:

In another embodiment the amplified product is bound to particles which are used for photothermal PCR and optical detection by extension of a primer with which said beads are functionalized. In this embodiment step (a) comprises the following steps:
(i) contacting the nucleotide target sequence with photothermal particles which are optically detectable and are functionalized with an oligonucleotide primer which is complementary to at least part of the target sequence;
(ii) allowing amplification of the target sequence in order to generate the amplified product;
(iii) exciting the photothermal particles to generate heat;
wherein the photothermal particles which are optically detectable are also used for optical detection in step (c).

Thus specific embodiments refer to methods comprising the following steps:
(a) photothermal PCR amplification of the nucleotide target sequence using photothermal particles functionalized with an oligonucleotide primer which is complementary to at least part of the target sequence to generate an amplified product; comprising the following steps:
   (i) contacting the nucleotide target sequence with photothermal particles which are optically detectable and are functionalized with an oligonucleotide primer which is complementary to at least part of the target sequence;
   (ii) allowing amplification of the target sequence in order to generate the amplified product;
   (iii) exciting the photothermal particles to generate heat;
(b) hybridization of the amplified product to a detection surface functionalized with an oligonucleotide probe complementary to at least part of the target sequence;
(c) measuring the amplified product by optically detecting optically detectable particles bound to the amplified product at the detection surface;

Step (a) maybe repeated for 1 to 100 cycles, preferably for 1 to 50 cycles, more preferably for 1 to 40 cycles, even more preferably for 1 to 30 cycles.

Also the methods measuring an amplified product which is bound to particles used for photothermal PCR and optical detection by extension of a primer with which said beads are functionalized can be carried out as end point detection, real-time detection or semi real-time detection assay. A schematic overview of one embodiment of the method in which the same particles are used for photothermal PCR amplification and for optical detection is shown in Figure 4.

The particles employed for both the PCR amplification step and the detection step are capable for excitation by electromagnetic radiation, in particular by excitation by light, in particular laser light and are capable for optical detection. In some embodiments these particles may be also magnetic which allows movement of the particles to the detection surface by magnetic forces.

### 3.1 Binding of the amplified product to particles used for photothermal PCR and optical detection by extension of a primer with which the particles are functionalized (endpoint assay)

In some embodiments the method employing the same particles for photothermal PCR and optical detection are carried out as end-point assays and comprises the following steps:
(a) photothermal PCR amplification of the nucleotide target sequence using photothermal particles functionalized with an oligonucleotide primer which is complementary to at least part of the target sequence to generate an amplified product; comprising the following steps:
   (i) contacting the nucleotide target sequence with photothermal particles which are optically detectable and are functionalized with an oligonucleotide primer which is complementary to at least part of the target sequence;
   (ii) allowing amplification of the target sequence in order to generate the amplified product;
   (iii) exciting the photothermal particles to generate heat;
(b) hybridization of the amplified product to a detection surface functionalized with an oligonucleotide probe complementary to at least part of the target sequence;
(c) measuring the amplified product by optically detecting optically detectable particles bound to the amplified product at the detection surface;
wherein step (a) is repeated for 1 to 100 cycles, preferably for 1 to 50 cycles, more preferably for 1 to 40 cycles, even more preferably for 1 to 30 cycles and wherein step (c) is carried out once after all repetitions of step (a) and step (b) are completed or alternatively wherein steps (b) and (c) are carried out once after all repetitions of step (a) are completed.

### 3.2 Binding of the amplified product to particles used for photothermal PCR and optical detection by extension of a primer with which the particles are functionalized (real-time assay)

In some embodiments the method employing the same particles for photothermal PCR and optical detection are carried out as real-time assays and comprises the following steps:
(a) photothermal PCR amplification of the nucleotide target sequence using photothermal particles functionalized with an oligonucleotide primer which is complementary to at least part of the target sequence to generate an amplified product; comprising the following steps:
   (i) contacting the nucleotide target sequence with photothermal particles which are optically detectable and are functionalized with an oligonucleotide primer which is complementary to at least part of the target sequence;
   (ii) allowing amplification of the target sequence in order to generate the amplified product;
   (iii) exciting the photothermal particles to generate heat;
(b) hybridization of the amplified product to a detection surface functionalized with an oligonucleotide probe complementary to at least part of the target sequence;
(c) measuring the amplified product by optically detecting optically detectable particles bound to the amplified product at the detection surface;
wherein steps (a) to (c) are repeated for 1 to 100 cycles, preferably for 1 to 50 cycles, more preferably for 1 to 40 cycles, even more preferably for 1 to 30 cycles.

### 3.2 Binding of the amplified product to particles used for photothermal PCR and optical detection by extension of a primer with which the particles are functionalized (semi-real-time assay)

In some embodiments the method employing the same particles for photothermal PCR and optical detection are carried out as semi-real-time assays and comprises the following steps:
(a) photothermal PCR amplification of the nucleotide target sequence using photothermal particles functionalized with an oligonucleotide primer which is complementary to at least part of the target sequence to generate an amplified product; comprising the following steps:
   (i) contacting the nucleotide target sequence with photothermal particles which are optically detectable and are functionalized with an oligonucleotide primer which is complementary to at least part of the target sequence;
   (ii) allowing amplification of the target sequence in order to generate the amplified product;
   (iii) exciting the photothermal particles to generate heat;
(b) hybridization of the amplified product to a detection surface functionalized with an oligonucleotide probe complementary to at least part of the target sequence;
(c) measuring the amplified product by optically detecting optically detectable particles bound to the amplified product at the detection surface;
wherein step (a) is repeated for 1 to 100 cycles, preferably for 1 to 50 cycles, more preferably for 1 to 40 cycles, even more preferably for 1 to 30 cycles and wherein step (c) is repeated with a lower frequency compared to step (a). Step (b) may be carried out in the frequency of step (a) or in the frequency of step (c).

### 4. Double labeling with magnetic particles for detection

Some embodiments refer to methods in which the photothermal particles are used which are functionalized with an oligonucleotide primer complementary to at least part of the target sequence and are functionalized with a binding molecule capable of binding a second binding molecule with which the optically detectable particles are functionalized. The binding molecule may be any binding molecule suitable for the specific binding of the photothermal particle and the optically detectable particle, it may be for example a nucleic acid probe or any specifically interacting molecules, for example peptide- antibody, biotin-streptavidin. In some embodiments, it may be beneficial that the non-covalent bond of the first and the second binding molecule have a high thermal stability which withstands the heat produces by the excitation of the photothermal particles.

In according embodiments step (a) comprises the following steps:
(i) contacting the nucleotide target sequence with
   - photothermal particles functionalized with a first oligonucleotide primer which is complementary to at least part of the target sequence and functionalized with a first binding molecule, and
   - optionally a second oligonucleotide primer which is complementary to at least part of the target sequence;
(ii) allowing amplification of the target sequence in order to generate the amplified product;
(iii) exciting the photothermal particles to generate heat;
(iv) contacting the photothermal particles with optically detectable particles functionalized with a second binding molecule capable of binding the first binding molecule of the photothermal particles;
Therefore, some embodiments of the invention may comprise the steps of
(a) photothermal PCR amplification of the nucleotide target sequence using photothermal particles functionalized with an oligonucleotide primer which is complementary to at least part of the target sequence to generate an amplified product;
   comprising the step of
   (i) contacting the nucleotide target sequence with
      - photothermal particles functionalized with a first oligonucleotide primer which is complementary to at least part of the target sequence and functionalized with a first binding molecule, and
      - optionally a second oligonucleotide primer which is complementary to at least part of the target sequence;
   (ii) allowing amplification of the target sequence in order to generate the amplified product;
   (iii) exciting the photothermal particles to generate heat;
(b) hybridization of the amplified product to a detection surface functionalized with an oligonucleotide probe complementary to at least part of the target sequence and contacting the photothermal particles with optically detectable particles functionalized with a second binding molecule capable of binding the first binding molecule of the photothermal particles;
(c) measuring the amplified product by optically detecting optically detectable particles bound to the amplified product at the detection surface.

Step (a) maybe repeated for 1 to 100 cycles, preferably for 1 to 50 cycles, more preferably for 1 to 40 cycles, even more preferably for 1 to 30 cycles.

The methods employing photothermal particles are used which are functionalized with an oligonucleotide primer complementary to at least part of the target sequence and are functionalized with a binding molecule capable of binding a second binding molecule with which the optically detectable particles are functionalized can be carried out as end point detection, real-time detection or semi real-time detection assay.

### 4.1 Endpoint assays

Exemplary embodiments that are carried out as end-point assays and comprises the following steps are:
(a) photothermal PCR amplification of the nucleotide target sequence using photothermal particles functionalized with an oligonucleotide primer which is complementary to at least part of the target sequence to generate an amplified product; comprising the step of
   (i) contacting the nucleotide target sequence with
      - photothermal particles functionalized with a first oligonucleotide primer which is complementary to at least part of the target sequence and functionalized with a first binding molecule, and
      - optionally a second oligonucleotide primer which is complementary to at least part of the target sequence;
   (ii) allowing amplification of the target sequence in order to generate the amplified product;
   (iii) exciting the photothermal particles to generate heat;
(b) hybridization of the amplified product to a detection surface functionalized with an oligonucleotide probe complementary to at least part of the target sequence and contacting the photothermal particles with optically detectable particles functionalized with a second binding molecule capable of binding the first binding molecule of the photothermal particles;
(c) measuring the amplified product by optically detecting optically detectable particles bound to the amplified product at the detection surface;
wherein step (a) is repeated for 1 to 100 cycles, preferably for 1 to 50 cycles, more preferably for 1 to 40 cycles, even more preferably for 1 to 30 cycles and wherein step (c) is carried out once after all repetitions of step (a) and step (b) are completed or alternatively wherein steps (b) and (c) are carried out once after all repetitions of step (a) are completed.

Figure 5 shows an exemplary embodiment of an endpoint assay which comprises the steps of
(a) photothermal PCR amplification of the nucleotide target sequence using gold particles functionalized with an oligonucleotide primer which is complementary to at least part of the target sequence to generate an amplified product; comprising the step of
   (i) contacting the nucleotide target sequence with
      - gold particles functionalized with a first oligonucleotide primer which is complementary to at least part of the target sequence and functionalized with a first binding molecule, and
      - optionally a second oligonucleotide primer which is complementary to at least part of the target sequence;
   (ii) allowing amplification of the target sequence in order to generate the amplified product;
   (iii) exciting the gold particles to generate heat;
(b) hybridization of the amplified product to a detection surface functionalized with an oligonucleotide probe complementary to at least part of the target sequence and contacting the photothermal particles with optically detectable magnetic particles functionalized with a second binding molecule capable of binding the first binding molecule of the photothermal particles;
(c) measuring the amplified product by optically detecting optically detectable particles bound to the amplified product at the detection surface;
wherein step (a) is repeated for 1 to 100 cycles, preferably for 1 to 50 cycles, more preferably for 1 to 40 cycles, even more preferably for 1 to 30 cycles and wherein steps (b) and (c) are carried out once after all repetitions of step (a) are completed.

### 4.2 Real-time assay

Exemplary embodiments that are carried out as real-time assays and comprises the following steps are:
(a) photothermal PCR amplification of the nucleotide target sequence using photothermal particles functionalized with an oligonucleotide primer which is complementary to at least part of the target sequence to generate an amplified product; comprising the step of
   (i) contacting the nucleotide target sequence with
      - photothermal particles functionalized with a first oligonucleotide primer which is complementary to at least part of the target sequence and functionalized with a first binding molecule, and
      - optionally a second oligonucleotide primer which is complementary to at least part of the target sequence;
   (ii) allowing amplification of the target sequence in order to generate the amplified product;
   (iii) exciting the photothermal particles to generate heat;
(b) hybridization of the amplified product to a detection surface functionalized with an oligonucleotide probe complementary to at least part of the target sequence and contacting the photothermal particles with optically detectable particles functionalized with a second binding molecule capable of binding the first binding molecule of the photothermal particles;
(c) measuring the amplified product by optically detecting optically detectable particles bound to the amplified product at the detection surface;
wherein steps (a) to (c) are repeated for 1 to 100 cycles, preferably for 1 to 50 cycles, more preferably for 1 to 40 cycles, even more preferably for 1 to 30 cycles.

### 4.3 Semi-real-time assay

Exemplary embodiments that are carried out as real-time assays and comprises the following steps are:
(a) photothermal PCR amplification of the nucleotide target sequence using photothermal particles functionalized with an oligonucleotide primer which is complementary to at least part of the target sequence to generate an amplified product; comprising the step of
   (i) contacting the nucleotide target sequence with
      - photothermal particles functionalized with a first oligonucleotide primer which is complementary to at least part of the target sequence and functionalized with a first binding molecule, and
      - optionally a second oligonucleotide primer which is complementary to at least part of the target sequence;
   (ii) allowing amplification of the target sequence in order to generate the amplified product;
   (iii) exciting the photothermal particles to generate heat;
(b) hybridization of the amplified product to a detection surface functionalized with an oligonucleotide probe complementary to at least part of the target sequence and contacting the photothermal particles with optically detectable particles functionalized with a second binding molecule capable of binding the first binding molecule of the photothermal particles;
(c) measuring the amplified product by optically detecting optically detectable particles bound to the amplified product at the detection surface;
wherein step (a) is repeated for 1 to 100 cycles, preferably for 1 to 50 cycles, more preferably for 1 to 40 cycles, even more preferably for 1 to 30 cycles and wherein step (c) is repeated with a lower frequency compared to step (a). Step (b) may be carried out in the frequency of step (a) or in the frequency of step (c).

### EXAMPLE

### Preparation of microfluidic system

The assay is carried out in a plastic microfluidic chamber with a depth of about 200 um and lateral dimensions 2 x 2 mm². The chamber bottom is polished to optical surface quality and is situated between two prisms that allow light to strike the surface at an angle above the critical angle of ca. 67°. The chamber top is formed by a transparent laminate of about 100 um thickness. The microfluidic chamber is connected with a transport channel that carries the liquid sample and is terminated by a fluidic valve that allows air to escape from the chamber while preventing the flow of liquid (e.g. a hydrophobic membrane). The laminate side of the chamber is contacted by a thermally conductive aluminum body whose temperature can be controlled by a thermoelectric element and a resistive heater to provide a temperature of up to 95°C in the reaction chamber. The polished side of the reaction chamber is in close proximity to the air gap of a horseshoe-like electromagnet that can be used to manipulate the magnetic particles inside the chamber and aid their binding to the detection surface. At the opposite side of the chamber, a solenoid washing magnet is located behind the heating plate at a distance that allows manipulation of the particles inside the reaction chamber. A laser source is positioned to emit a laser beam to enter the detection chamber via its polished bottom surface. A mirror galvanometer in the beam path provides the possibility to scan the laser beam across the bottom of the detection chamber.

### Preparation of gold nanoparticles

Gold nanoparticles of a mean diameter of 30 nm are functionalized with thiol-modified forward primers.

### Preparation of magnetic detection particles

Magnetic detection particles are functionalized by EDC/NHS coupling of 5'-amino-modified nucleic acid probes. The probe sequence is chosen such that it overlaps with the sequence of the generated amplicon, but neither of the primer sequences.

### Execution of amplification

Amplification is carried out by bringing a mixture of both particles into the microfluidic chamber. To achieve initial melting of the double-stranded DNA target, the temperature in the detection chamber is increased to 95°C for about 10 seconds before being reduced to 50°C for the remaining time of the amplification reaction. At the same time, the washing magnet is powered to attract all magnetic particles to the top surface of the reaction chamber to minimize interplay between the magnetic particles and the gold nanoparticles and to avoid interactions between the magnetic particles and the excitation laser. After reducing the temperature of the reaction chamber to 50°C, the laser focus is scanned across the bottom of the reaction chamber to achieve intermittent heating of the gold nanoparticles in the sample. In this process, the target sequence is amplified. The reaction products are bound to the gold nanoparticles and are freely available in solution. In regular intervals, the amplification process is interrupted by switching off the laser to allow for detection of the generated amplification products.

### Detection

Detection is achieved by first switching off the washing magnet and allowing the magnetic detection particles to diffuse for a 20 seconds and to bind unbound amplicons. After this capturing phase, the particles are attracted to the detection surface by the horseshoe magnets. When close to the surface, the magnetic field is pulsed to create phases of magnetic attraction interspersed with phases of free diffusion to ensure efficient binding of magnetic particles to binding spots on the surface. After 30 seconds the horseshoe magnets are turned off and the washing magnet is used to attract all unbound particles back to the top of the reaction chamber. The amount of bound particles is determined by surface-sensitive FTIR-based detection and is a measure for the currently available concentration of amplicons in the sample.

## Claims

1. A method for amplification and optical detection of a nucleotide target sequence comprising the following steps:
(a) photothermal PCR amplification of the nucleotide target sequence using photothermal particles functionalized with an oligonucleotide primer which is complementary to at least part of the target sequence to generate an amplified product;
(b) hybridization of the amplified product to a detection surface functionalized with an oligonucleotide probe complementary to at least part of the target sequence;
(c) measuring the amplified product by optically detecting optically detectable particles bound to the amplified product at the detection surface;
wherein the amplified product is allowed to bind to the optically detectable particles during and/or after the photothermal PCR amplification.

2. The method according to claim 1, wherein the optically detectable particles are magnetic particles and step (b) further comprises moving the magnetic particles to the detection surface using magnetic forces.

3. The method according to claim 1 or 2, wherein steps (a) to (c) are carried out in the same reaction chamber, wherein the reaction chamber is preferably part of a microfluidic cartridge.

4. The method according to any one of the previous claims, wherein the photothermal particles have a size of 5 nm to 150 nm, preferably of 10 nm to 100 nm, more preferably of 20 nm to 90 nm and most preferably of 30 nm to 80 nm.

5. The method according to any one of the previous claims, wherein the photothermal particles are gold particles.

6. The method according to any one of the previous claims, wherein step (a) comprises exciting the photothermal particles to generate heat.

7. The method according to claim 6, wherein the exciting is achieved by exposing the photothermal particles to laser light.

8. The method according to any one of the previous claims, wherein the detecting in step (c) is performed using frustrated total internal reflection (FTIR).

9. The method according to any one of the previous claims, wherein the oligonucleotide probe of the detection surface is blocked at the 3'-end.

10. The method according to any one of the previous claims, wherein step (a) comprises the following steps:
(i) contacting the nucleotide target sequence with
- photothermal particles functionalized with a first oligonucleotide primer which is complementary to at least part of the target sequence, and
- a second oligonucleotide primer which is complementary to at least part of the target sequence;
(ii) allowing amplification of the target sequence in order to generate the amplified product;
(iii) exciting the photothermal particles to generate heat;
wherein the amplified product is allowed to bind to optically detectable particles by means of hybridization to an oligonucleotide probe which is complementary to at least part of the target sequence and with which the optically detectable particles are functionalized.

11. The method according to any one claims 1 to 9, wherein step (a) comprises the following steps:
(i) contacting the nucleotide target sequence with
- photothermal particles functionalized with a first oligonucleotide primer which is complementary to at least part of the target sequence, and
- optically detectable particles functionalized with a second oligonucleotide primer which is complementary to at least part of the target sequence;
(ii) allowing amplification of the target sequence in order to generate the amplified product;
(iii) exciting the photothermal particles to generate heat;

12. The method according to any one of claims 1 to 9, wherein step (a) comprises the following steps:
(i) contacting the nucleotide target sequence with photothermal particles which are optically detectable and are functionalized with an oligonucleotide primer which is complementary to at least part of the target sequence;
(ii) allowing amplification of the target sequence in order to generate the amplified product;
(iii) exciting the photothermal particles to generate heat;
wherein the photothermal particles which are optically detectable are also used for optical detection in step (c).

13. The method according to any one of claims 1 to 9, wherein step (a) comprises the following steps:
(i) contacting the nucleotide target sequence with
- photothermal particles functionalized with a first oligonucleotide primer which is complementary to at least part of the target sequence and functionalized with a first binding molecule, and
- optionally a second oligonucleotide primer which is complementary to at least part of the target sequence;
(ii) allowing amplification of the target sequence in order to generate the amplified product;
(iii) exciting the photothermal particles to generate heat;
(iv) contacting the photothermal particles with optically detectable particles functionalized with a second binding molecule capable of binding the first binding molecule of the photothermal particles;

14. The method according to any one of claims 1 to 13, wherein step (a) is repeated for 1 to 100 cycles, preferably for 1 to 50 cycles, more preferably for 1 to 40 cycles, even more preferably for 1 to 30 cycles.

15. The method according to claim 14, wherein step (b) and/or (c) are not repeated or are repeated.

16. The method according to claim 15, wherein steps (b) and/or (c) are repeated with the same or with different frequencies compared to step (a).

17. The method according to claim 16, wherein the frequency of step (a) is higher than the frequency of step (c).
